(19) **Europäisches Patentamt / European Patent Office / Office européen des brevets**

(11) **EP 4 725 494 A1**

(12) **EUROPEAN PATENT APPLICATION**
published in accordance with Art. 153(4) EPC

(43) Date of publication:
**15.04.2026 Bulletin 2026/16**

(21) Application number: **24822919.7**

(22) Date of filing: **11.06.2024**

(51) International Patent Classification (IPC):
**A61K 36/23** (2006.01)     **A61P 11/00** (2006.01)
**A61P 11/06** (2006.01)     **A23L 33/105** (2016.01)

(52) Cooperative Patent Classification (CPC):
**A61P 11/00; A61K 36/23;** A61K 2236/17;
A61K 2236/333; A61K 2236/50

(86) International application number:
**PCT/IB2024/055679**

(87) International publication number:
**WO 2024/256954 (19.12.2024 Gazette 2024/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **11.06.2023 US 202363507468 P**
**25.07.2023 US 202363515341 P**

(71) Applicant: **Efil Bioscience Inc.**
**Daejeon 34165 (KR)**

(72) Inventors:
• **KIM, Jeong-Hoon**
  **Daejeon 34165 (KR)**
• **CHOI, Mu Lim**
  **Daejeon 34165 (KR)**
• **KIM, Kongsik**
  **Daejeon 34165 (KR)**

(74) Representative: **You Patent**
**3rd & 4th Floor**
**84 Salop Street**
**Wolverhampton WV3 0SR (GB)**

(54) **COMPOSITION AND METHOD FOR TREATING, PREVENTING, AND ALLEVIATING RESPIRATORY SYMPTOM OR DISEASE**

(57)     The present invention provides a method for preparing a processed parsnip extract having improved physiological activity compared to a raw parsnip extract, which comprises pretreating raw parsnip to produce an aged parsnip and extracting the aged parsnip. Furthermore, the present invention provides a composition comprising the parsnip extract and a method for promoting respiratory health and/or preventing, alleviating, or treating respiratory symptoms or diseases by using the composition.

EP 4 725 494 A1

**Description**

## TECHNICAL FIELD

The present invention relates to methods for preparing parsnip extracts with enhanced physiological activities, compositions comprising the parsnip extracts prepared by the methods, and methods for promoting respiratory health by using the compositions.

## BACKGROUND ART

Mammals, including humans, suffer from various respiratory symptoms and diseases due to genetic or environmental factors, and there is an urgent need for innovative solutions to effectively prevent, improve, or treat these respiratory symptoms or diseases.

As reported in, e.g., J Clin Invest, 111: 291-297, 2003; N Engl J. Med. 2001; 44(5): 350-62; Toshio Hirano, Cytokine molecular biology, World Science, 2002; Am J Respir Crit Care Med, 2013, 187: 347-365; Am J Respir Crit Care Med, 2009, 180:396-406; Biol Pharm Bull, 2012, 35:1752-1760; Am J Respir Crit Care Med, 1997 155, 1441-1447; Am J Physiol Lung CellMol Physiol, 2010, 298:L262-L269; Am J Respir Cell Mol Biol, 2013, 48:531-539; Eur Respir J, 1998, 12:1200-1208; LoS One. 2014 May 15; 9(5): e97784; Respir Res. 2015 May 22; 16:59.; J Immunol Res. 2017; 2017:6710278; Respirology. 2016 Apr; 21(3): 467-75; and Am J Respir Crit Care Med 154(1): 76-81, 1996, which are incorporated herein by reference, research on bronchial asthma and chronic obstructive pulmonary disease is actively underway. However, there are few functional foods or drugs to effectively prevent, improve, or treat bronchial asthma and chronic obstructive disease.

## SUMMARY

The present invention provides a method for pre-treating raw parsnips into aged parsnips and extracting these aged parsnips to produce parsnip extracts having physiological activities higher than those of raw parsnips. Also, it provides a composition comprising the parsnip extracts having enhanced physiological activities. In addition, it provides a method for promoting respiratory health and/or preventing, improving, or treating respiratory symptoms or diseases by using the composition.

**In** one aspect, the present invention provides a composition for promoting respiratory health. More specifically, in one aspect, the present invention provides a composition for promoting respiratory health, the composition comprising processed parsnip extracts having a total polyphenol content greater than that of unprocessed raw (natural) parsnips. The promotion of respiratory health may include preventing, improving, or treating diseases, disorders, conditions, or symptoms associated with respiratory diseases. Respiratory diseases may include bronchial asthma or chronic obstructive pulmonary disease. The processed parsnip extracts of the present invention may preferably have a total polyphenol content of at least 1.5 times, preferably at least 2 times, and mor preferably at least 3 times more than the raw parsnips.

**In** accordance with an embodiment of the present invention, raw parsnips are heat-dried at a predetermined range of temperature (or a predetermined temperature) for a predetermined period of time (or a predetermined time) to the point where the color of the raw parsnips changes to brown or black, and then the extraction process is applied to obtain the extract. As an example, the predetermined range of temperature can be adjusted to a temperature between about 50°C and about 70°C, and the predetermined period can be adjusted to a period between about 10 and about 30 days. As another example, the predetermined range of temperature can be adjusted to a temperature between about 55°C and about 65°C, and the predetermined c period can be adjusted to a period between about 10 and about 20 days. As another example, the predetermined range of temperature can be adjusted to a temperature between about 70°C and about 90°C, and the predetermined period can be adjusted to a period between about 3 and about 10 days. As another example, the predetermined range of temperature can be adjusted to a temperature between about 85°C and about 90°C, and the predetermined period can be adjusted to a period between 3 and 10 days. According to some other embodiments, the predetermined range of temperature and the predetermined period of time can be adjusted to such an extent that the color of the raw parsnips changes to brown or black and/or that the total polyphenol content of the processed parsnips becomes greater than the total polyphenol content of the raw parsnips.

In another aspect, the present invention provides a method for preparing the extract. More specifically, in another aspect, the present invention provides a method of preparing processed aged parsnip extracts having a total polyphenol content greater than (e.g., at least 2 times greater than) that of unprocessed raw (natural) parsnips. The preparation method includes the steps of heat drying raw parsnips for a predetermined period of time at a predetermined range of temperature, adding alcohol, water, or a mixture thereof to the thus heat-dried parsnips, filtering the supernatant after extraction, and removing unnecessary components. In accordance with an embodiment of the present invention, the heat drying step may be conducted to such an extent that the color of the raw parsnips changes to brown or black and that the total polyphenol content of the processed parsnips becomes greater than the total polyphenol content of the raw parsnips.

The above and other aspects of the invention and representative embodiments of the invention are described in more detail below.

## BRIEF DESCRIPTION OF DRAWINGS

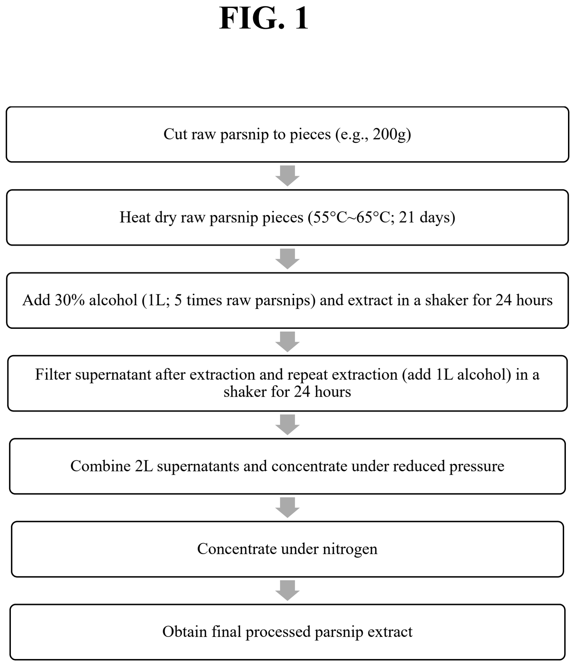

FIG. 1 illustrates a process of preparing parsnip extracts in accordance with an embodiment of the present invention.

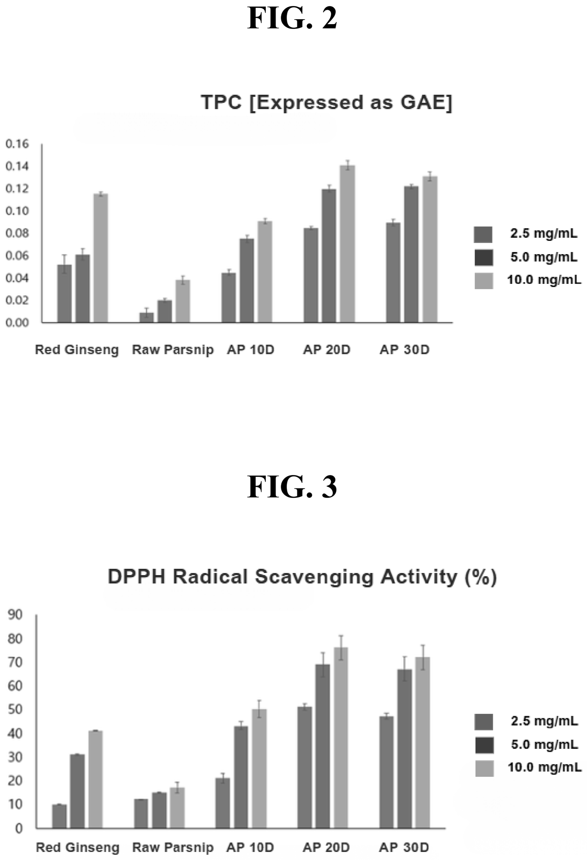

FIG. 2 is a graph representing the change of total polyphenol content of the parsnip extract of the embodiment of the present invention. AP 10D, AP 20D, and AP 30D refer to processed parsnip extracts obtained by pretreating (alternatively, aging or processing) for 10 days, 20 days, and 30 days, respectively, and Raw Parsnip refers to parsnip extract obtained without conducting such pretreatment.

FIG. 3 is a graph showing the 2,2-diphenyl-1-picrylhydrazyl (DPPH) radical scavenging activity of the parsnip extract in accordance with the embodiment of the present invention. AP 10D, AP 20D, and AP 30D refer to processed parsnip extracts obtained by pretreating (alternatively, aging or processing) for 10 days, 20 days, and 30 days, respectively, and Raw Parsnip refers to parsnip extract obtained without conducting such pretreatment.

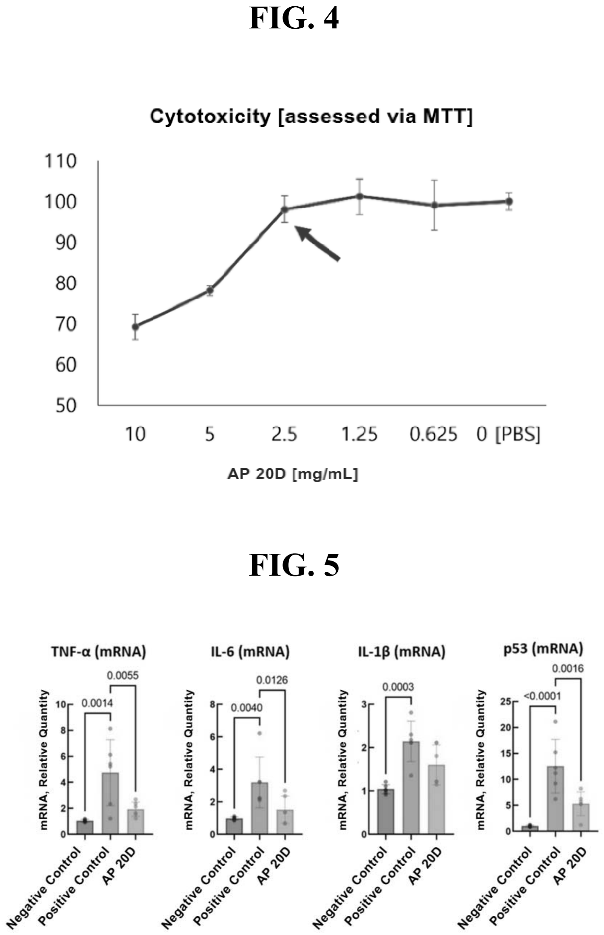

FIG. 4 is a graph showing the cytotoxicity of the parsnip extract in accordance with the embodiment of the present invention in human bronchial epithelial cells (BEAS-2B). AP 20D refers to processed parsnip extracts obtained by pretreating (alternatively, aging or processing) for 20 days. MTT refers to 2,5-diphenyl-2H-tetrazoliumbromide. PBS refers to phosphate buffered saline.

FIG. 5 shows the effect of the parsnip extract in accordance with the embodiment of the present invention from acrolein-induced inflammation in human bronchial epithelial cells (BEAS-2B). NEG refers to a negative control, POS refers to a positive control to which acrolein alone was treated, AP 20D refers to a test group to which acrolein and the parsnip extract obtained by pretreating (alternatively, aging or processing) for 20 days were treated. P values were calculated by step-wise t-test.

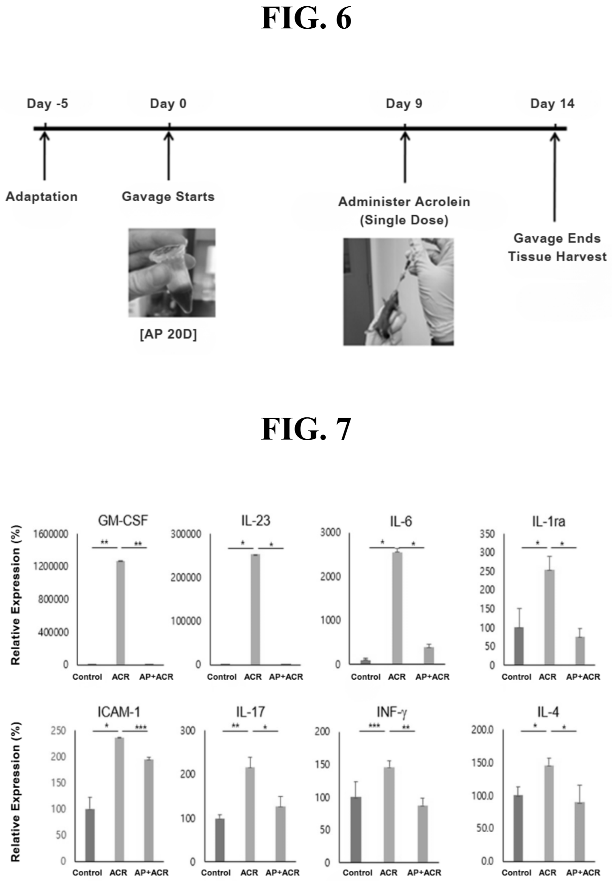

FIG. 6 illustrates an experimental design to evaluate the anti-inflammatory effect of the parsnip extract in accordance with the embodiment of the invention on mice exposed to acrolein. Control refers to a negative control group to which basal diet (AIN-93G) was treated. ACR refers to a positive control group to which the basal diet and acrolein were treated. AP+ACR refers to a test group to which the basal diet, acrolein, and the parsnip extract obtained by pretreating (alternatively, aging or processing) for 20 days in accordance with the embodiment of the invention were treated.

FIG. 7 shows the effect of the parsnip extract in accordance with the embodiment of the invention on expression of blood inflammatory cytokines. CONT refers to a negative control group to which basal diet (AIN-93G) was treated. ACR refers to a positive control group to which the basal diet and acrolein were treated. AP+ACR refers to a test group to which the basal diet, acrolein, and the parsnip extract obtained by pretreating (alternatively, aging or processing) for 20 days in accordance with the embodiment of the invention were treated. * refers to $P<0.05$, ** refers to $P<0.01$, and *** refers to $P<0.001$. P values were calculated by step-wise t-test.

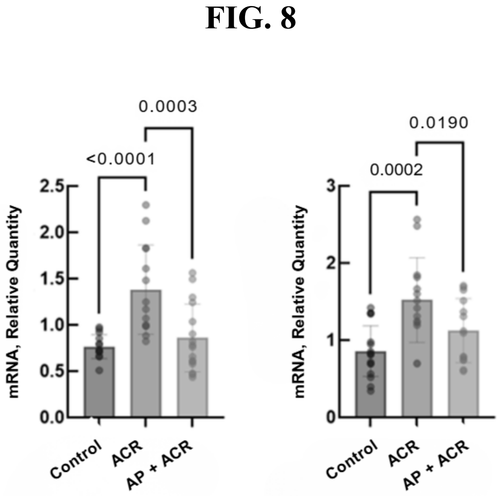

FIG. 8 shows the effect of the parsnip extract in accordance with the embodiment of the invention on expression of TNF-$\alpha$ mRNA and IL-1$\beta$ mRNA. CONT refers to a negative control group to which basal diet (AIN-93G) was treated. ACR refers to a positive control group to which the basal diet and acrolein were treated. AP+ACR refers to a test group to which the basal diet, acrolein, and the parsnip extract obtained by pretreating (alternatively, aging or processing) for 20 days in accordance with the embodiment of the invention were treated. P values were calculated by step-wise t-test.

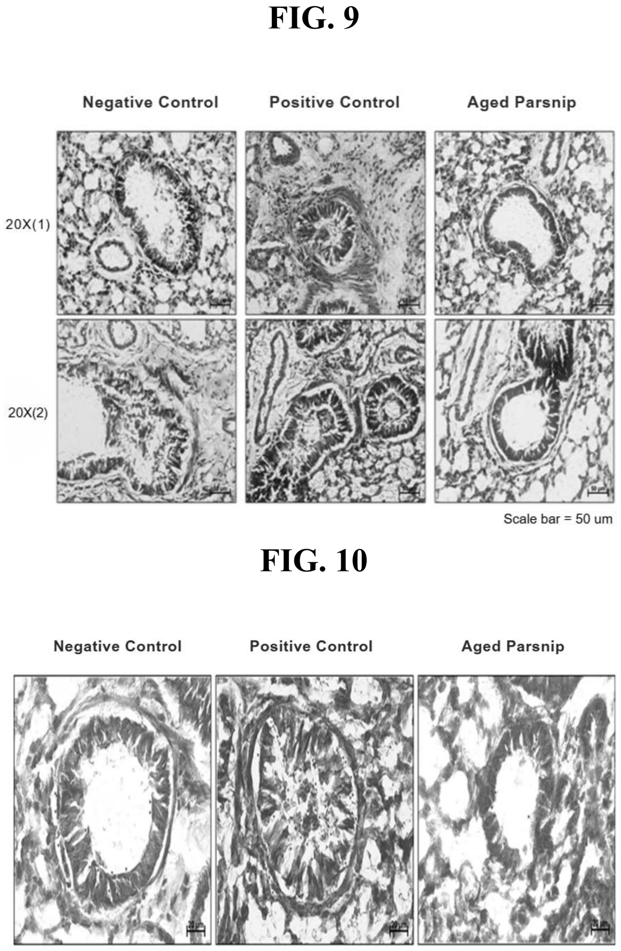

FIG. 9 shows the H&E staining result of a lung tissue treated with the parsnip extract in accordance with the embodiment of the invention. Negative refers to a negative control group to which basal diet (AIN-93G) was treated. Positive refers to a positive control group to which the basal diet and acrolein were treated. Aged Parsnip refers to a test group to which the basal diet, acrolein, and the parsnip extract obtained by pretreating (alternatively, aging or processing) for 20 days in accordance with the embodiment of the invention were treated.

FIG. 10 shows the PAS staining result of a lung tissue treated with the parsnip extract in accordance with the embodiment of the invention. Negative refers to a negative control group to which basal diet (AIN-93G) was treated. Positive refers to a positive control group to which the basal diet and acrolein were treated. Aged Parsnip refers to a test group to which the basal diet, acrolein, and the parsnip extract obtained by pretreating (alternatively, aging or processing) for 20 days in accordance with the embodiment of the invention were treated.

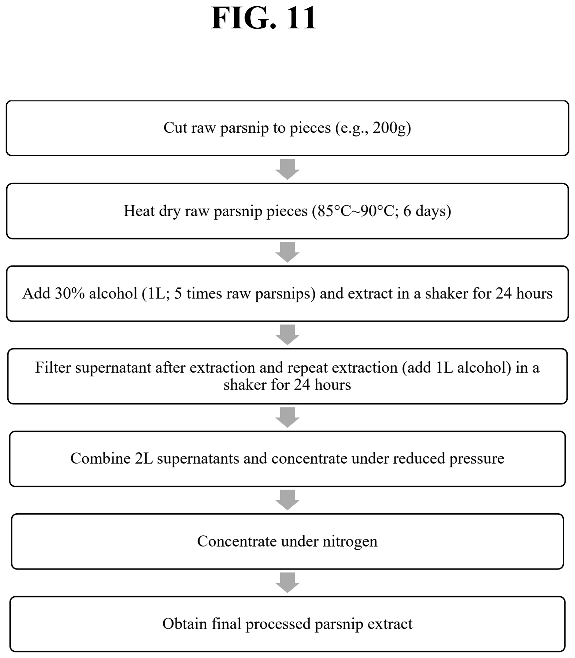

FIG. 11 illustrates a process of preparing parsnip extracts in accordance with another embodiment of the present invention.

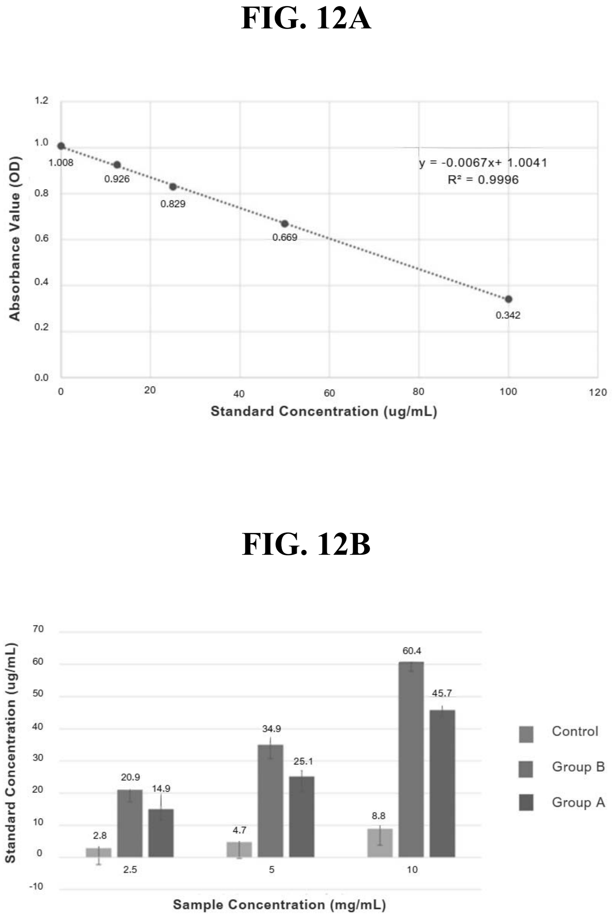

FIG. 12A is the standard (calibration) line used to measure the DPPH radical scavenging activity of the parsnip extract according to the another embodiment of the present invention, and FIG.12B is a graph showing the DPPH radical scavenging activity of the parsnip extract sample according to the another embodiment of the present invention. Group A refers to a test group to which the parsnip extract prepared in Example 1 of the present invention, Group B refers to a test group to which the parsnip extract prepared in Example 6 of the present invention, and CONT refers to a control group to which extracts obtained from raw parsnips without performing the pretreatment (aging) process.

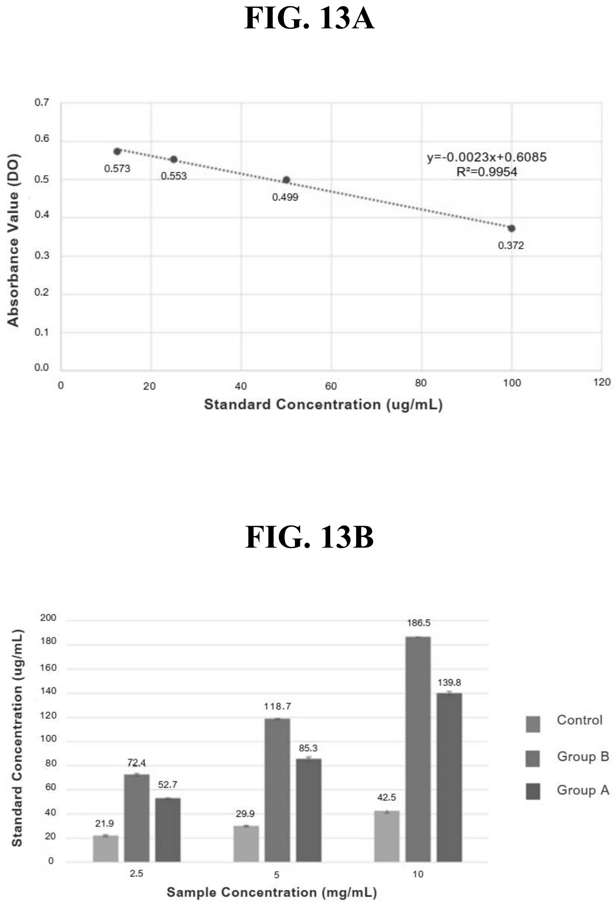

FIG. 13A is the standard line used to measure the ABTS radical scavenging activity of the parsnip extract according to the another embodiment of the present invention, and FIG. 13B is a graph representing the ABTS radical scavenging activity of the parsnip extract according to the another embodiment of the present invention. Group A refers to a test group to which the parsnip extract prepared in Example 1 of the present invention, Group B refers to a test group to which the parsnip extract prepared in Example 6 of the present invention, and CONT refers to a control group to which extracts obtained from raw parsnips without performing the pretreatment (aging) process.

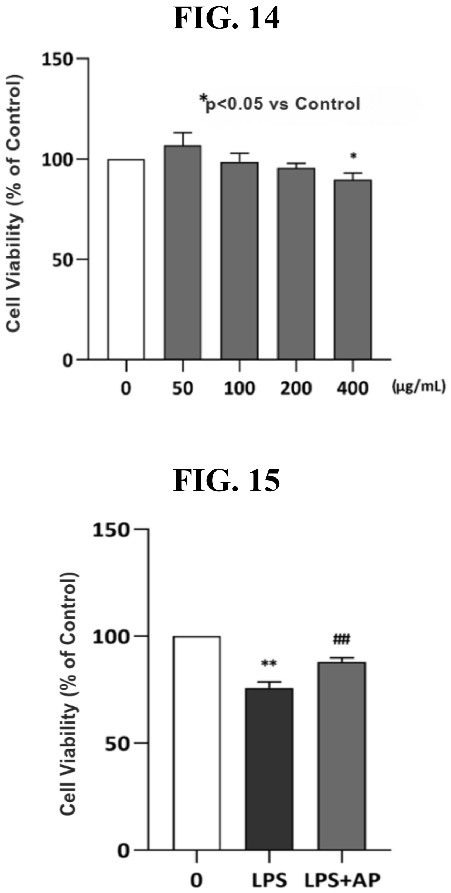

FIG. 14 shows the cytotoxicity of the parsnip extract in accordance with the another embodiment of the invention on human primary bronchial/tracheal epithelial cells.

FIG. 15 shows the effect of the parsnip extract in accordance with the another embodiment of the invention to protect lung tissue.

## DETAILED DESCRIPTION

For the purpose of promoting an understanding of the principles of the present disclosure, reference will now be made to the embodiments illustrated in the drawings, and specific language will be used to describe the same. It will nevertheless be understood that no limitation of the scope of this disclosure is thereby intended.

In the present disclosure the term "about" can allow for a degree of variability in a value or range, for example, within 10%, within 5%, or within 1% of a stated value or of a stated limit of a range.

In the present disclosure the term "substantially" can allow for a degree of variability in a value or range, for example, within 90%, within 95%, or within 99% of a stated value or of a stated limit of a range.

In this document, the terms "a," "an," or "the" are used to include one or more than one unless the context clearly dictates otherwise.

The term "or" is used to refer to a nonexclusive "or" unless otherwise indicated.

In addition, it is to be understood that the phraseology or terminology employed herein, and not otherwise defined, is for the purpose of description only and not of limitation. Any use of section headings is intended to aid reading of the document and is not to be interpreted as limiting. Further, information that is relevant to a section heading may occur within or outside of that section. Furthermore, all publications, patents, and patent documents referred to in this document are incorporated by reference herein in their entirety, as though individually incorporated by reference. In the event of inconsistent usages between this document and those documents so incorporated by reference, the usage in the incorporated references should be considered supplementary to that of this document; for irreconcilable inconsistencies, the usage in this document controls.

## I. PARSNIP EXTRACTS

In one aspect, the present invention provides parsnip extracts having physiological activities greater than raw parsnips. In accordance with an embodiment of the prevent invention, raw parsnips are pretreated to make aged or processed parsnips, and the aged or processed parsnips are subjected to an extraction process, thereby preparing the parsnip extracts of the invention. The parsnip extracts of the invention have physiological activities greater than raw parsnips.

A process of pretreating raw parsnips was reported in Plant Foods for Human Nutrition (2020) 75:292-297), but the process takes too much time (4 weeks) to complete and requires high temperature (80°C) heat treatment and a high level of humidity (95%), which limits its application in industrial sites. There is an urgent need for a new method of pre-treating raw parsnips with a shorter period of time and milder process conditions.

According to embodiments of the present invention, it is possible to obtain parsnip extracts with significantly improved physiological activities compared to raw parsnips by pre-treating raw parsnips with mild process conditions and/or for a relatively short time.

In accordance with embodiments of the invention, raw parsnips are pretreated to make aged parsnips. The raw parsnips can be cut into pieces before pretreatment. The shape (e.g., round, spherical, cuboid, cube, etc.) and size (e.g., large, medium, small, etc.) can be adjusted accordingly as needed. During the pretreatment process, temperature, time, and/or pressure can be set appropriately.

For example, the temperature can suitably be heated to about 40~90°C, 45~85°C, 50~80°C, 55~75°C, or 60~70°C. However, the above-listed ranges are only examples, and the temperature can be adjusted accordingly as needed. The heating can be suitably conducted for a predetermined period of time. For example, it can be heated for about 1~30 days, 5~25 days, 10~20 days, or 13~17 days. However, the above-listed ranges are only illustrative, and the heating period can be adjusted accordingly as needed. The pressure condition can suitably be adjusted appropriately during the heating. For example, it can be done under atmospheric pressure conditions or at high pressure. However, the above pressure conditions are only examples, and the pressure conditions can be adjusted accordingly as needed. For example, the heating and drying process can be conducted either wet or dry. If necessary, wet drying and dry drying can be used together.

After the pretreatment, water, alcohol, or a mixture thereof is added for extraction. The type of alcohol can be appropriately selected according to needs. Preferably, ethanol may be chosen. The concentration of alcohol can be adjusted as needed. Preferably, about 10~45% alcohol can be used. Preferably, about 15~40% alcohol can be used. Even more preferably, 20~30% alcohol can be used. However, these alcohol concentration ranges are only illustrative and can be adjusted as needed. Preferably, water, alcohol, or a water/alcohol mixture can be used in greater amount than the raw parsnips (e.g., 2, 3, 4, 5, 6, ...times raw parsnips).

Thereafter, the aged parsnips are extracted in a stirring shaker. The temperature and time for the extraction can be selected according to needs. For example, they can be extracted at room temperature for 24 hours. Supernatants are filtered after the extraction, and additional extraction can be conducted, if needed. All supernatants obtained in the extraction process are collected, and the collected supernatants are concentrated under reduced pressure to remove residual organic solvents and treated with nitrogen to remove residual moisture.

## II. COMPOSITIONS

In another aspect, the present invention provides a composition comprising the parsnip extracts having physiological activities greater than raw parsnips for use in preventing, improving (alleviating), or treating respiratory diseases, disorders, conditions, or symptoms.

As used herein, the term "composition" is intended to contain the extract obtained by the above-described methods or contain any other product resulting directly or indirectly from the extract. The term "composition" is intended to contain a combination of at least one of the extract and at least one of the product resulting from the extract, a combination of two or more of the extracts, or a combination of two or more of the products resulting from the extracts.

As used herein, the term "composition" refers to a mixture of a pharmaceutically or therapeutically active component (ingredient) with one or more other components, which may be chemically or biologically active or inactive. Such components may include, but not limited to, carriers, stabilizers, diluents, dispersing agents, suspending agents, thickening agents, excipients, and adjuvants.

Any suitable pharmaceutically acceptable carriers, stabilizers, diluents, dispersing agents, suspending agents, thickening agents, excipients, and adjuvants known to those of ordinary skill in the art for use in pharmaceutical compositions may be selected and employed in the compositions described herein. As used herein, the term "acceptable" with respect to a formulation, composition, or ingredient, as used herein, means having no persistent detrimental effect on the general health of the subject being treated. As used herein, the term "carrier" refers to chemical or biological material that can facilitate the incorporation of a therapeutically active ingredient(s) into cells or tissues. Examples of the pharmaceutically acceptable carrier include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, and the like, but it is not limited thereto.

Suitable excipients may include, for example, water, pharmaceutically acceptable organic solvents such as paraffins (e.g., petroleum fractions), vegetable oils (e.g. groundnut or sesame oil), mono- or polyfunctional alcohols (e.g., ethanol or glycerol), carriers such as natural mineral powders (e.g., kaoline, clays, talc, chalk), synthetic mineral powders (e.g., highly dispersed silicic acid and silicates), sugars (e.g., cane sugar, lactose and glucose), emulsifiers (e.g., lignin, spent sulphite liquors, methylcellulose, starch and polyvinylpyrrolidone), and lubricants (e.g., magnesium stearate, talc, stearic acid and sodium lauryl sulphate).

The compositions described herein may be in the form of a solid, liquid, or gas (aerosol). For example, they may be in the form of tablets (coated tablets) made of, for example, collidone or shellac, gum Arabic, talc, titanium dioxide or sugar, capsules (gelatin), solutions (aqueous or aqueous-ethanolic solution), syrups containing the active substances, emulsions or inhalable powders (of various saccharides such as lactose or glucose, salts and mixture of these excipients with one another), and aerosols (propellant-containing or -free inhale solutions). Also, the compositions described herein may be formulated for sustained or slow release.

## III. METHODS

In still another aspect, the present invention provides a method of using a composition comprising the parsnip extracts having enhanced physiological activities to promote respiratory health and/or to prevent, improve, or treat respiratory symptoms, conditions, or diseases.

As used herein, the term "treat," "treating" or "treatment" refers to methods of alleviating, abating, ameliorating, inhibiting, relieving, stopping, or preventing a disease, disorder, condition, or symptom. The term also refers to methods of alleviating, abating, ameliorating, inhibiting, relieving, stopping, or preventing the underlying causes of the disease, disorder, condition, or symptom.

As used herein, the term "subject" or "patient" encompasses mammals and non-mammals. Examples of mammals include, but are not limited to, humans, chimpanzees, apes monkeys, cattle, horses, sheep, goats, swine; rabbits, dogs, cats, rats, mice, guinea pigs, and the like. Examples of non-mammals include, but are not limited to, birds, fishes and the like.

As used herein, the term "administration" or "administering" of the subject composition refers to providing a composition of the invention and/or a prodrug thereof to a subject in need of treatment.

As used herein, the term "effective amount" or "therapeutically effective amount" refer to a sufficient amount of an active ingredient(s) described herein being administered which will, for example, relieve to some extent one or more of the symptoms of the disease or condition being treated. The result can be reduction and/or alleviation of the signs, symptoms, or causes of a disease, or any other desired alteration of a biological system. For example, an "effective amount" for therapeutic uses is the amount of the composition as disclosed herein required to provide a clinically significant decrease in disease symptoms. An appropriate "effective" amount in any individual case may be determined using techniques, such as a dose-escalation study.

In addition, such compositions may be administered singly or in combination with one or more additional functional or therapeutic agents. The methods of administration of such compositions may include, but are not limited to, intravenous administration, inhalation, oral administration, rectal administration, parenteral, intravitreal administration, subcutaneous administration, intramuscular administration, intranasal administration, dermal administration, topical administration, ophthalmic administration, buccal administration, tracheal administration, bronchial administration, sublingual administration or optic administration. The method of administration of the composition is determined according to the degree of symptoms. In addition, the dosage of the active ingredient in the composition may vary depending on the route of administration, the severity of the disease, the age, the sex, and weight of the patient, and may be administered once to several times a day.

Compositions provided herein may be administered by way of known formulations, including tablets, capsules or elixirs for oral administration, suppositories for rectal administration, sterile solutions or suspensions for parenteral or intramuscular administration, lotions, gels, ointments or creams for topical administration, and the like. In some embodiments, such compositions are formulated as tablets, pills, capsules, a liquid, an inhalant, a nasal spray solution, a suppository, a solution, a gel, an emulsion, an ointment, eye drops, or ear drops.

The therapeutically effective amount may vary depending on, among others, the disease indicated, the severity of the disease, the age and relative health of the subject, the potency of the compound administered, the mode of administration and the treatment desired. The required dosage will also vary depending on the mode of administration, the particular condition to be treated and the effect desired.

The above and other embodiments and uses will be apparent to one skilled in the art in light of the present disclosures. The following examples are provided merely as illustrative of various embodiments and shall not be construed to limit the invention in any way.

## EXAMPLES

Hereinafter, the present invention will be described in more detail through examples. These examples are only intended to illustrate the present invention in more detail, and it will be apparent to those skilled in the art that the scope of the present invention is not limited by these examples according to the gist of the present invention.

**Example** 1: **Preparation of Parsnip Extract Sample A**

Raw parsnips were peeled, washed, and cut to pieces so that each piece was about 200g. The parsnip pieces were placed in a drying chamber maintained at 55°C~65°C and heat-dried for at least 10 days. 30% alcohol (or a mixture of alcohol and water) was added to the heat-dried parsnip pieces. The extraction was performed at room temperature (20°C ~25°C) for 24 hours in a stirred incubator (125 rpm). The supernatant was filtered by a filter paper (Whatman No 4). The parsnips remaining on the filter paper were extracted one more time under the same conditions, and the supernatant was collected one more time. The thus-obtained final processed parsnip extract was subjected to reduced pressure extraction to remove residual organic solvents and then subjected to nitrogen treatment to remove residual moisture. The preparation process is illustrated in Figure 1.

**Example 2: Evaluation of Antioxidant Properties of Parsnip Extract Sample A**

**1. Determination of Total Polyphenol Content**

2.5 mg/mL, 5.0 mg/mL, and 10.0 mg/mL of the final processed parsnip extracts prepared in Example 1 were prepared. 100 μL of 2.5 mg/mL of the final processed parsnip extract and 200 μL of 2% Na2CO3 were reacted for 3 minutes, 100 μL of 5.0 mg/mL of the final processed parsnip extract and 200 μL of 2% Na2CO3 were reacted for 3 minutes, and 100 μL of 10.0 mg/mL of the final processed parsnip extract and 200 μL of 2% Na2CO3 were reacted for 3 minutes. To the resulting mixtures, 10 μL of 50% Folin-ciocalteu reagent was added and reacted for 3 minutes, and then absorbance was measured at 720 nm. As for a blank, the organic solvent (i.e., 15% DMSO), which was used to dissolve parsnip extract, was mixed and reacted with 2% Na2CO3 for 3 minutes. For the control, 50% Folin-ciocalteu reagent was added to the blank (i.e., the mixture of 15% DMSO and 2% Na2CO3) and reacted for 3 minutes. The content of total polyphenols was shown against a calibration curve using gallic acid.

As shown in Figure 2, processed parsnips had a total polyphenol content that was approximately twice as high as raw parsnips as early as day 10, and under the 20-day and 30-day processing conditions, the total polyphenol content was equivalent to or higher than that of red ginseng, depending on the concentration. There was no significant difference between processed parsnips manufactured under 20-day and 30-day processing conditions. Taken together, the total polyphenol content of processed parsnips prepared by the method of the present invention was significantly higher than the total polyphenol content of raw parsnips (Figure 2). For example, the total polyphenol content of the processed parsnip obtained by processing 20 or longer days was similar to or greater than that of commercially available red ginseng. Processed parsnips with such an elevated level of total polyphenol content could be obtained in a fairly short time.

**2. DPPH Radical Scavenging Activity**

To evaluate the antioxidant properties of the processed parsnips, the DPPH radical scavenging test was performed in addition to the analysis of the total polyphenol content. 2.5 mg/mL, 5.0 mg/mL, and 10.0 mg/mL of the final processed parsnip extracts prepared in Example 1 were prepared. 20 μL of 2.5 mg/mL of the final processed parsnip extract and 80 μL of 0.3 mM DPPH (2,2-diphenyl-1-picrylhydrazyl) reagent were reacted for 1 hour under dark condition, 20 μL of 5.0 mg/mL of the final processed parsnip extract and 80 μL of 0.3 mM DPPH (2,2-diphenyl-1-picrylhydrazyl) reagent were reacted for 1 hour under dark condition, and 20 μL of 10.0 mg/mL of the final processed parsnip extract and 80 μL of 0.3 mM DPPH (2,2-diphenyl-1-picrylhydrazyl) reagent were reacted for 1 hour under dark condition. Then, absorbance was measured at 720 nm. As for a blank optical density, 20uL of the organic solvent (i.e., 10% DMSO), which was used to dissolve parsnip extract, was mixed with 80 uL of 100% ethanol for 1 hour, and as for the control optical density, 20uL of the organic solvent (i.e., 10% DMSO) was mixed with 0.3 mM DPPH (80 uL) and then reacted for 1 hour. Antioxidant activity was calculated by the following formula.

$$\text{Antioxidant activity (\%)} = 100 \times \left( 1 - \frac{\text{Sample OD} - \text{Blank OD}}{\text{Control OD} - \text{Blank OD}} \right)$$

As shown in Figure 3, the processed parsnips showed about three times higher DPPH radical scavenging activity compared to raw parsnips as early as day 10 (in case of 5.0 mg/mL and 10 mg/mL), and the processed parsnips obtained under the 20-day and 30-day processing conditions showed higher DPPH radical scavenging activities than red ginseng. Similar to the total polyphenol content analysis, there was no significant difference between processed parsnips prepared under 20-day and 30-day processing conditions. Taken together, the DPPH radical scavenging activity of the processed parsnips prepared by the method of the present invention was significantly higher than that of raw parsnips. For example, the DPPH scavenging activity of the processed parsnip obtained by processing twenty or longer days was significantly greater than that of commercially available red ginseng (Figure 3). Processed parsnips with such an elevated level of DPPH scavenging activity could be obtained in a fairly short time.

**Example 3: Effect of Processed Parsnip Extract Sample A to Treat, Alleviate, and Prevent Respiratory Inflammation (In Vitro Study)**

**1. Cytotoxicity Assay**

Based on the results of Example 2, it was observed that after a certain period of time (e.g., 20 days), the physiological activity of the processed parsnip did not increase linearly even if additional dry heating processing was conducted. Experiments were conducted to establish the maximum concentration of processed parsnips (20-day aged parsnips) that do not cause cytotoxicity. To verify that the processed parsnips have inhibitory effect on respiratory inflammation, BEAS-2B human bronchial epithelial cells were cultured at 37°C and 5% $CO_2$ saturation. The cultured cells are dispensed at a 96-well place with conditions of $5 \times 10^4$ cells/cm2 for cytotoxicity experiments and stabilized for 12 hours, and the processed parsnips (here, 20-day processed parsnips) were treated at various concentrations. More specifically, the concentrations were 0 mg/mL (in this case, phosphate buffered saline was treated instead of the processed parsnip), 0.625 mg/mL, 1.25 mg/mL, 2.5 mg/mL, 5 mg/mL, and 10 mg/mL. After 24 hours, cell viability was determined by MTT assay, and the highest concentration (here, 2.5 mg/mL) that did not affect the cell viability was determined (Figure 4).

**2. Effect of Processed Parsnip Extract to Treat, Alleviate, and Prevent Respiratory Inflammation Induced by Acrolein**

Acrolein is one of the indicators of air pollution and is a highly reactive aldehyde family. It is known to not only cause oxidative stress in cells but also bind to various macromolecules (e.g., DNA or proteins) and induce inflammatory responses and apoptosis. Based on the results of the above-mentioned study, it was evaluated at the cellular level whether parsnip extract processed for 20 days under non-toxic treatment conditions had a significant preventive effect on acrolein-induced inflammation. More specifically, the processed parsnip extract of the present invention was treated to human bronchial epithelial cells (BEAS-2B cells), and then 160 $\mu$M of acrolein was treated. Gene expression in the signaling system associated with inflammation was analyzed. As shown in Figure 5, in the group to which acrolein alone was treated, expression of both markers of inflammation-related cytokines (TNF-$\alpha$, IL-6, IL-1$\beta$) and p53 involved in apoptosis were significantly increased. Conversely, in the group treated with the processed parsnip extracts (2.5 mg/mL), the inflammatory and apoptosis responses were significantly reduced (Figure 5).

Similarly, it was also verified at the cellular level whether the processed parsnip extract of the present invention has a significant effect of treating acrolein-induced inflammation under non-toxic treatment conditions. More specifically, 160 $\mu$M of acrolein was treated to BEAS-2B human bronchial epithelial cells, and then the processed parsnip extracts were treated. Gene expression of signaling systems involved in inflammation was analyzed. Although not shown here, we could obtain results similar to Figure 5.

**Example 4: Effect of Processed Parsnip Extract Sample A to Treat, Alleviate, and Prevent Respiratory Inflammation (In Vivo Study)**

**1. Inflammatory Markers**

Based on the results of the above cell experiments, C57BL6 mice were used to verify the effect of the processed parsnip on markers of respiratory inflammation. More specifically, C57BL6 male mice were orally administered with 10 mg/kg bw of the processed parsnip extract in accordance with the present invention for 2 weeks. From day 9, acrolein was nasally administered for 5 days (nasal installation; 10 $\mu$g/kg bw). On day 14, the animals were sacrificed, and inflammatory markers were analyzed (Figure 6).

**2. Cytokine Protein Content**

The protein content of various pro-inflammatory cytokines contained in the blood samples obtained from the test animal was analyzed. The inflammatory cytokines in the blood shown in Figure 7 are generally known to be markers closely related to anti-inflammatory mechanisms. As shown in Figure 7, the expression of inflammatory cytokines induced by acrolein was significantly reduced by treatment with the extract of parsnip (Figure 7).

## 3. Inflammatory Gene Expression in Lung Tissue

In addition to the systemic inflammatory response identified in Figure 7, to analyze the local inflammatory response, lung tissue was extracted from the mice, and the expression levels of TNF-$\alpha$ and IL-1$\beta$, two genes that are key to the inflammatory response, were measured. The anti-inflammatory effect of the processed parsnip extract was identified in the local lung inflammatory response test (Figure 8).

## 4. H&E Staining Assay for Lung Tissue

Histological analysis of the lung tissue was performed by H&E staining. As shown in Figure 9, bronchial lesions in lung tissue were induced to a significant extent in the positive control group compared to the negative control group, but the occurrence of bronchial lesions in the lung tissue was reduced in the group treated with the processed parsnip extracts in accordance with embodiments of the present invention.

## 5. PAS Staining Assay for Lung Tissue

The lung tissue was PAS stained to determine the degree of mucus accumulation in the lung tissue. As shown in Figure 10, mucus accumulation in the lung tissue of the positive control group significantly increased compared to the negative control group, but mucus accumulation in the lung tissue of the test group significantly decreased.

As in the cell model study, in the animal model study, it was verified whether the processed parsnip extract of the present invention at a concentration not causing toxicity has a significant effect to treat acrolein-induced inflammation. More specifically, acrolein was first treated to C57BL6 male mice, the processed parsnip extract was then treated, and inflammatory markers were analyzed. Although not shown here, we could obtain results similar to those shown in Figures 7 through 10.

## Example 6: Preparation of Parsnip Extract Sample B

As in Example 1, raw parsnips were peeled, washed, and cut to pieces so that each piece was about 200g. The parsnip pieces were placed in a drying chamber maintained at 85~90°C and heat-dried for 6 days. 30% alcohol (or a mixture of alcohol and water) was added to the heat-dried parsnip pieces. The extraction was performed at room temperature (20 ~25°C) for 24 hours in a stirred incubator (125 rpm). The supernatant was filtered by a filter paper (Whatman No 4). The parsnips remaining on the filter paper were extracted one more time under the same conditions, and the supernatant was collected one more time. The thus-obtained final processed parsnip extract was subjected to reduced pressure extraction to remove residual organic solvents and then subjected to nitrogen treatment to remove residual moisture. The preparation process is illustrated in Figure 11.

## Example 7: Evaluation of Antioxidant Properties of Parsnip Extract Samples A and B

### 1. DPPH Radical Scavenging Activity

To evaluate the antioxidant properties of the processed parsnips, the DPPH radical scavenging test was performed. 2.5 mg/mL, 5.0 mg/mL, and 10.0 mg/mL of the final processed parsnip extracts prepared in Example 6 were prepared. 20 $\mu$L of 2.5 mg/mL of the final processed parsnip extract and 180 $\mu$L of 0.2 mM DPPH (2,2-diphenyl-1-picrylhydrazyl) reagent were reacted for 1 hour under dark condition, 20 $\mu$L of 5.0 mg/mL of the final processed parsnip extract and 180 $\mu$L of 0.2 mM (2,2-diphenyl-1-picrylhydrazyl) reagent were reacted for 1 hour under dark condition, and 20 $\mu$L of 10.0 mg/mL of the final processed parsnip extract and 180 $\mu$L of 0.2 mM (2,2-diphenyl-1-picrylhydrazyl) reagent were reacted for 30 minutes under dark condition. Then, absorbance was measured at 517 nm.

For the control, vitamin C (0~100 ug/mL) was reacted with DPPH reagent under the same conditions. Using the measured absorbance value, a standard line (Figure 12 A) was obtained, and the DPPH radical scavenging activity of the processed parsnip extract of the present invention was measured by reading against the vitamin C data. As shown in Figure 12B, the DPPH radical scavenging activity of the processed parsnip extract sample A according to the present invention and the DPPH radical scavenging activity of the processed parsnip extract sample B according to the present invention were superior to the DPPH radical scavenging activity of the raw material parsnip extract that had not undergone aging treatment.

### 2. ABTS Radical Scavenging Activity

The antioxidant activity of the processed parsnip extracts was assayed by using 2,2'-azino-bis(3-ethylbenzothiazoline-6-sulfonic acid (ABTS) and 2,2-Azobis(2-methylpropionamidine)dihydrochloride (AAPH). ABTS was dissolved in 100 mL of PBS at a concentration of 2.5 mM, heated at 70°C for 1 hour, and cooled at room temperature for 20~30 minutes. AAPH was dissolved in 100 mL of PBS at a concentration of 1 mM, heated at 70°C for 1 hour, and cooled at room temperature for 20~30 minutes. As a standard solution, vitamin C was prepared by continuous dilution from 100 $\mu$g/mL to 0 $\mu$g/mL. The sample solution was continuously diluted so that the absorbance at 734 nm could be between 0.65 and 0.9. 10 $\mu$L of the standard solution and 10 $\mu$L of the sample solution respectively were placed in a 96-well plate, 190 $\mu$L of ABTS solution was added, and the mixture was reacted at room temperature for 10 minutes. Based on the absorbance data read at 734 nm, the radical scavenging activity of the sample was calculated by reading against the vitamin C concentration by using the standard equation (Figure 13A). As shown in Figure 13B, the ABTS radical scavenger ability of the processed parsnip extract sample A according to the present invention and the ABTS radical scavenging ability of the processed parsnip extract sample B according to the present invention were superior to the ABTS radical scavenger ability of the raw parsnip extract that has not been aged.

**Example 8: Effect of Processed Parsnip Extract Sample B to Treat, Alleviate, and Prevent Respiratory Inflammation (In Vitro Study)**

**1. Cytotoxicity Assay**

To verify whether the antioxidant activity of processed parsnip extracts according to the present invention acts as a protective effect on lung cells, the efficacy test was conducted by using human primary bronchial/tracheal epithelial cells. The cells were cultured in 37°C, 5% $CO_2$ environment with 10% FB S and 1% penicillin-streptomycin in DMEM/F-12 medium. The cells were inoculated in a 96-well plate at a density of $5 \times 10^4$ cells/well and were grown for 24 hours. The parsnip extracts at various concentrations (0, 25, 50, 100, 200, and 400 $\mu$g/mL) were prepared, 100 $\mu$L of the respective parsnip extractions were added to the well, and the cells were incubated for 24 hours. MTT solution was prepared in PBS at a concentration of 5 mg/mL, 10 $\mu$L of the MTT solution was added to each well to make the final concentration of 0.5 mg/mL, and the cells were incubated for 4 hours. The medium was removed, and 100 $\mu$L of DMSO was added to dissolve the formazan crystals. Absorbance was measured at 570 nm, and background absorbance at 630 nm was calibrated. Cell viability was calculated as a percentage compared to the control group. All experiments were repeated three times. Data were expressed as mean $\pm$ standard deviation. Statistical analysis was performed using GraphPad Prism using one-way analysis of variance (ANOVA) and Tukey's post-hoc test. A p-value of less than 0.05 was considered statistically significant. As shown in Figure 14, the processed parsnip extract sample B according to the present invention showed cytotoxicity at a concentration of 400 ug/mL.

**2. Effect of Processed Parsnip Extract to Protect Lung Cell from Inflammation Induced by Acrolein**

Experiments were conducted with the maximum concentration of processed parsnips that do not cause cytotoxicity. 20 ug/mL of LPS was administered to a group for 24 hours to induce inflammation. The processed parsnip extract sample B and LPS were treated to the other group. After 24 hours, cell viability was determined by MTT assay (Figure 15). As shown in Figure 15, the processed parsnip extract B according to the present invention protected human lung cells from inflammation induced by LPS.

Similarly, it was also verified at the cellular level whether the processed parsnip extract of the present invention has a significant effect of treating LPS-induced inflammation under non-toxic treatment conditions. More specifically, LPS was treated to human primary bronchial/tracheal epithelial cells, and then the processed parsnip extracts were treated. Gene expression of signaling systems involved in inflammation was analyzed. Although not shown here, we could obtain results similar to Figure 15.

**Claims**

1.   A composition for promoting respiratory health, which comprises a processed parsnip extract having a total polyphenol content greater than that of unprocessed raw parsnip.

2.   The composition of claim 1, wherein said promoting of respiratory health includes preventing, improving, or treating disorders, conditions, or symptoms associated with a respiratory disease.

3.   The composition of claim 2, wherein the respiratory disease is bronchial asthma.

4.   The composition of claim 2, wherein the respiratory disease is chronic obstructive pulmonary disease.

5. The composition of claim 1, wherein the processed parsnip extract is prepared by pretreating raw parsnip for a predetermined period of time at a predetermined range of temperature until the color of the raw parsnip changes to brown or black to make aged parsnip and extracting the aged parsnip.

6. The composition of claim 5, wherein the predetermined temperature is in the range of 50°C to 65°C and the predetermined time is in the range of 10 to 20 days.

7. The composition of claim 5, wherein the predetermined temperature is in the range of 85°C to 90°C and the predetermined time is in the range of 3 to 10 days.

8. The composition of claim 1, wherein the processed parsnip extract has a total polyphenol content that is at least 2 times greater than that of the raw parsnip.

9. A method of preparing a processed parsnip extract having a higher antioxidant activity compared to unprocessed raw parsnip, the method comprising:

> heat-drying raw parsnip at a predetermined temperature for a predetermined time;
> adding alcohol, water, or a mixture thereof to the heat-dried raw parsnip;
> filtering a supernatant; and
> removing unnecessary components from the filtered supernatant.

10. The method of claim 9, wherein the heat-drying is conducted until the color of the raw parsnip changes to brown or black.

# FIG. 1

```
┌─────────────────────────────────────────────────────────────┐
│           Cut raw parsnip to pieces (e.g., 200g)             │
└─────────────────────────────────────────────────────────────┘
                              ▼
┌─────────────────────────────────────────────────────────────┐
│        Heat dry raw parsnip pieces (55°C~65°C; 21 days)      │
└─────────────────────────────────────────────────────────────┘
                              ▼
┌─────────────────────────────────────────────────────────────┐
│ Add 30% alcohol (1L; 5 times raw parsnips) and extract in a  │
│                   shaker for 24 hours                        │
└─────────────────────────────────────────────────────────────┘
                              ▼
┌─────────────────────────────────────────────────────────────┐
│ Filter supernatant after extraction and repeat extraction    │
│        (add 1L alcohol) in a shaker for 24 hours             │
└─────────────────────────────────────────────────────────────┘
                              ▼
┌─────────────────────────────────────────────────────────────┐
│ Combine 2L supernatants and concentrate under reduced        │
│                        pressure                              │
└─────────────────────────────────────────────────────────────┘
                              ▼
┌─────────────────────────────────────────────────────────────┐
│                 Concentrate under nitrogen                   │
└─────────────────────────────────────────────────────────────┘
                              ▼
┌─────────────────────────────────────────────────────────────┐
│            Obtain final processed parsnip extract            │
└─────────────────────────────────────────────────────────────┘
```

# FIG. 2

**TPC [Expressed as GAE]**

- 2.5 mg/mL
- 5.0 mg/mL
- 10.0 mg/mL

# FIG. 3

**DPPH Radical Scavenging Activity (%)**

- 2.5 mg/mL
- 5.0 mg/mL
- 10.0 mg/mL

# FIG. 4

**Cytotoxicity [assessed via MTT]**

# FIG. 5

# FIG. 6

# FIG. 7

# FIG. 8

# FIG. 9

Negative Control    Positive Control    Aged Parsnip

20X(1)

20X(2)

Scale bar = 50 um

# FIG. 10

Negative Control    Positive Control    Aged Parsnip

# FIG. 11

Cut raw parsnip to pieces (e.g., 200g)

Heat dry raw parsnip pieces (85°C~90°C; 6 days)

Add 30% alcohol (1L; 5 times raw parsnips) and extract in a shaker for 24 hours

Filter supernatant after extraction and repeat extraction (add 1L alcohol) in a shaker for 24 hours

Combine 2L supernatants and concentrate under reduced pressure

Concentrate under nitrogen

Obtain final processed parsnip extract

# FIG. 12A

# FIG. 12B

# FIG. 13A

# FIG. 13B

# FIG. 14

# FIG. 15

## INTERNATIONAL SEARCH REPORT

| International application No. |
|---|
| **PCT/IB2024/055679** |

| A. | CLASSIFICATION OF SUBJECT MATTER |
|---|---|
| | **A61K 36/23**(2006.01)i; **A61P 11/00**(2006.01)i; **A61P 11/06**(2006.01)i; **A23L 33/105**(2016.01)i |

According to International Patent Classification (IPC) or to both national classification and IPC

| B. | FIELDS SEARCHED |
|---|---|

Minimum documentation searched (classification system followed by classification symbols)

A61K 36/23(2006.01); A61K 36/736(2006.01); A61K 36/752(2006.01); A61K 36/8964(2006.01); A61K 36/899(2006.01); A61P 11/00(2006.01); A61P 11/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 파스닙(parsnip), 폴리페놀(polyphenol), 호흡기(respiratory), 천식(asthma), 폐질환 (lung disease), DPPH 라디칼(DPPH radical), ABTS 라디칼(ABTS radical)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KENARI, H. M. et al. Review of Pharmacological Properties and Chemical Constituents of Pastinaca sativa. Journal of Pharmacopuncture. 2021, vol. 24, no. 1, pp. 14-23. <br> See page 19; and tables 1 and 2. | 1-10 |
| A | CN 103495057 A (SHANDONG PROVINCIAL CENTER FOR ANIMAL DISEASE CONTROL AND PREVENTION et al.) 08 January 2014 (2014-01-08) <br> See claims 1 and 2. | 1-10 |
| A | CN 105535196 A (DALIAN DENT BIOTECHNOLOGY CO., LTD.) 04 May 2016 (2016-05-04) <br> See abstract; and claim 1. | 1-10 |
| A | CN 105596470 A (GUANGXI UNIVERSITY OF CHINESE MEDICINE PHARMACEUTICAL FACTORY) 25 May 2016 (2016-05-25) <br> See abstract; and claim 1. | 1-10 |
| A | CN 104083602 A (YANG, Liguo) 08 October 2014 (2014-10-08) <br> See claims 1-3. | 1-10 |

☐ Further documents are listed in the continuation of Box C.　　☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 September 2024** | **11 September 2024** |

| Name and mailing address of the ISA/KR | Authorized officer |
|---|---|
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/IB2024/055679**

| Patent document cited in search report | | | Publication date (day/month/year) | Patent family member(s) | | | Publication date (day/month/year) |
|---|---|---|---|---|---|---|---|
| CN | 103495057 | A | 08 January 2014 | CN | 103495057 | B | 10 December 2014 |
| | | | | CN | 201041522 | Y | 26 March 2008 |
| CN | 105535196 | A | 04 May 2016 | None | | | |
| CN | 105596470 | A | 25 May 2016 | None | | | |
| CN | 104083602 | A | 08 October 2014 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *J Clin Invest*, 2003, vol. 111, 291-297
- *N Engl J. Med.*, 2001, vol. 44 (5), 350-62
- **TOSHIO HIRANO**. Cytokine molecular biology. *World Science*, 2002
- *Am J Respir Crit Care Med*, 2013, vol. 187, 347-365
- *Am J Respir Crit Care Med*, 2009, vol. 180, 396-406
- *Biol Pharm Bull*, 2012, vol. 35, 1752-1760
- *Am J Respir Crit Care Med*, 1997, vol. 155, 1441-1447
- *Am J Physiol Lung CellMol Physiol*, 2010, vol. 298, L262-L269
- *Am J Respir Cell Mol Biol*, 2013, vol. 48, 531-539
- *Eur Respir J*, 1998, vol. 12, 1200-1208
- *LoS One.*, 15 May 2014, vol. 9 (5), e97784
- *Respir Res.*, 22 May 2015, vol. 16, 59
- *J Immunol Res*, 2017, vol. 2017, 6710278
- *Respirology.*, April 2016, vol. 21 (3), 467-75
- *Am J Respir Crit Care Med*, 1996, vol. 154 (1), 76-81
- *Plant Foods for Human Nutrition*, 2020, vol. 75, 292-297